# EUROPEAN PATENT APPLICATION

(11) **EP 4 737 569 A1**
(43) Date of publication of application: **06.05.2026**
(21) Application number: 24832371.9
(22) Date of filing: 21.06.2024
(51) Int. Cl.: C12N 15/11

(54) **C(5)-HALOGENATED PYRIMIDINE RIBONUCLEOTIDE-BOUND MRNA AND METHOD FOR INCREASING PROTEIN EXPRESSION USING SAME**

(30) Priority: 29.06.2023 KR 20230083892
(71) Applicant: INDUSTRIAL COOPERATION FOUNDATION JEONBUK NATIONAL UNIVERSITY, Jeonju-si, Jeollabuk-do 54896 (KR)
(72) Inventor: SEO, Young Jun, Jeonju-si, Jeonbuk-do 54970 (KR); CHABUNGBAM, Dhurbachandra Singh, Jeonju-si, Jeonbuk-do 54896 (KR); ALOM, Kazi Morshed, Jeonbuk-do 54896 (KR); THOKCHOM, Simander Singh, Jeonju-si, Jeonbuk-do 54896 (KR)
(74) Representative: Mewburn Ellis LLP
(86) International application number: PCT/KR2024/008625
(87) International publication number: WO 2025/005592

(57) **Abstract**

The present invention relates to mRNA to which C (5)-halogenated pyrimidine ribonucleotide is bound and a technique for increasing protein expression using same. Various C(5) halogenated pyrimidine (cytidine/uridine) ribonucleotides were synthesized and single- and double-base modified rNTPs were bound to cap-dependent and cap-independent mRNA and then examined for transcription and translation efficiency. As a result, the translation efficiency of fluorinated and chlorinated modified mRNA was 4-5 times higher than that of unmodified mRNA in the case of single base modification, and the combination of pseudouridine and 5-fluorocytidine was significantly increased by about 10 times or more compared to unmodified RNTP in the case of double base modification. Therefore, the present invention can be advantageously utilized in a treatment method requiring modified mRNA.

## Description

### TECHNICAL FIELD

The present invention relates to C(5)-halogenated pyrimidine ribonucleotide-bound mRNA and to a technique for increasing protein expression using the same.

### BACKGROUND ART

To treat the COVID-19 pandemic that has been spreading worldwide, mRNA vaccines have played a pivotal role. The main components of mRNA vaccines are modified nucleosides (pseudouridine, ψ) and methyl nucleosides (1-methylpseudouridine, m1ψ). The introduced mRNA can evade innate immune responses and express antigens with high efficiency, possesses many advantages such as rapid protein production, and avoids harmful chromosomal integration-a serious adverse effect of DNA-based vaccines. Accordingly, many research institutions have conducted preclinical and clinical studies for the therapeutic application of mRNA, and have demonstrated therapeutic efficacy using modified mRNA platforms.

Exogenous mRNA introduced into cells is recognized by various pattern recognition receptors (PRRs) present in endosomes or the cytosol, which induces activation of the type I interferon (IFN-I) pathway and causes an excessive immune response that inhibits expression of antigen proteins. However, it is known that mRNA into which chemically modified nucleosides-pseudouridine and 1-methylpseudouridine-are introduced can evade activation of TLR7, TLR8, and other innate immune sensors, thereby reducing type I interferon activation efficiency and increasing the efficiency of protein expression. Modified nucleosides are analogs other than the conventional A, U, G, and C; and mRNA into which such modified nucleic acids are introduced may exhibit slightly different secondary-structure modifications even for the same sequence, may not be recognized by RNA pattern recognition receptors so as to evade excessive immunity, and is also known to provide different efficiencies in terms of RNA structural stability.

Through research, it has been demonstrated that, by singly or doubly modifying bases such as N6-methyladenosine (m6A), pseudouridine (ψ), 5-methylcytidine (m5C), 2-thiouridine (s2U), and 5-methyluridine (m5U), the TLR-mediated immunogenicity of mRNA is suppressed, thereby lowering the immunogenicity of in vitro-transcribed mRNA; and by substituting uridine with pseudouridine, the stability of mRNA and high translational capacity have been demonstrated. These two research results were important endeavors that changed the perception and reality regarding mRNA and have recently contributed to the development of modified mRNA as therapeutics. In addition, for therapeutic applications of mRNA, many researchers have reported the effects of modified mRNA in various therapeutic fields using m5C/ψ or m5C/s2U-modified mRNA; and to utilize it in various therapeutic fields, it is important to develop new and diverse modified mRNA. However, the present inventors screened various commercially available purine- and pyrimidine-modified mRNAs for protein expression, but most did not exhibit efficient transcription and translation efficiency.

Accordingly, in an effort to identify modified nucleotides capable of increasing protein expression efficiency, the present inventors focused on C(5) modification of pyrimidines, among pyrimidine modifications, which can produce more stable mRNA due to higher in vivo stability and bioavailability. Thus, it was confirmed that, when modified nucleotides in which halogens are substituted at the fifth carbon of the cytidine and uridine pyrimidine bases are introduced into an mRNA construct, excessive immune responses are evaded and the expression efficiency of antigen proteins can be increased. In addition, unlike the usual decrease in transcription efficiency when using modified nucleotides, it was confirmed that, when modified nucleotides in which halogens are substituted at the fifth carbon of the cytidine and uridine pyrimidine bases were used, transcription efficiency did not decrease, and the present invention was completed.

### DETAILED DESCRIPTION OF THE INVENTION

### TECHNICAL PROBLEM

The problem to be solved by the present invention is to provide a method for increasing protein expression efficiency using an mRNA construct in which C(5)-halogenated pyrimidine ribonucleotides are incorporated.

### TECHNICAL SOLUTION

In order to solve the above-described technical problem, in one embodiment of the present invention, there is provided an mRNA construct into which a modified nucleotide, in which the fifth carbon of a pyrimidine base of cytidine or uridine is substituted with a halogen, is introduced.

In another embodiment, there is provided a composition for RNA synthesis comprising the modified nucleotide.

In an embodiment of the present invention, there is also provided a method for increasing protein expression efficiency using mRNA into which the modified nucleotide is introduced.

In the present invention, the composition for RNA synthesis may further comprise pseudouridine or methylpseudouridine.

In the present invention, the method may be characterized in that protein expression efficiency is increased through cap-dependent translation.

In the present invention, the method may further comprise pseudouridine or methylpseudouridine, and particularly may comprise 5-fluoro cytidine triphosphate (5-F rCTP) represented by Chemical Formula 1 and pseudouridine, wherein the protein may be an antigen protein.

The modified nucleotide may be selected from the group consisting of the following Chemical Formulas 1 to 4.

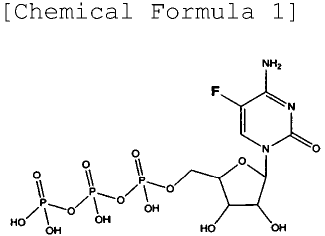

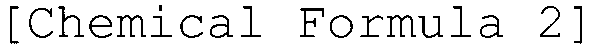

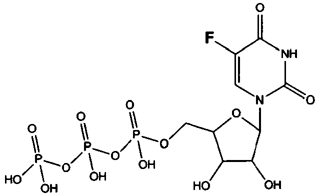

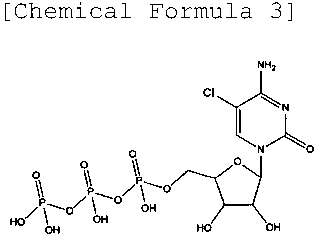

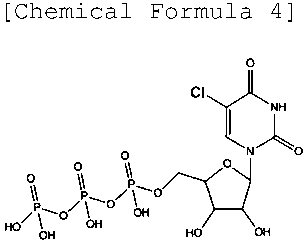

### ADVANTAGEOUS EFFECTS

As a result of synthesizing various C(5)-halogenated pyrimidine (cytidine/uridine) ribonucleotides and conjugating single- and double-base modified rNTPs to cap-dependent and cap-independent mRNA, and then confirming transcription and translation efficiencies, it was found that fluorinated and chlorinated modified mRNAs exhibited 4-5 times higher translation efficiency than unmodified mRNA in the case of single-base modification, and in the case of double-base modification, the combination of pseudouridine and 5-fluorocytidine showed an increase of about 10-fold or more compared with unmodified rNTPs. Accordingly, it is expected that the invention can be effectively utilized in therapeutic methods depending on modified mRNA.

### DESCRIPTION OF THE DRAWINGS

FIG. 1 is a schematic diagram showing the structures of purchased 5-fluorocytidine (1a) and 5-fluorouridine (2a) ribonucleosides.

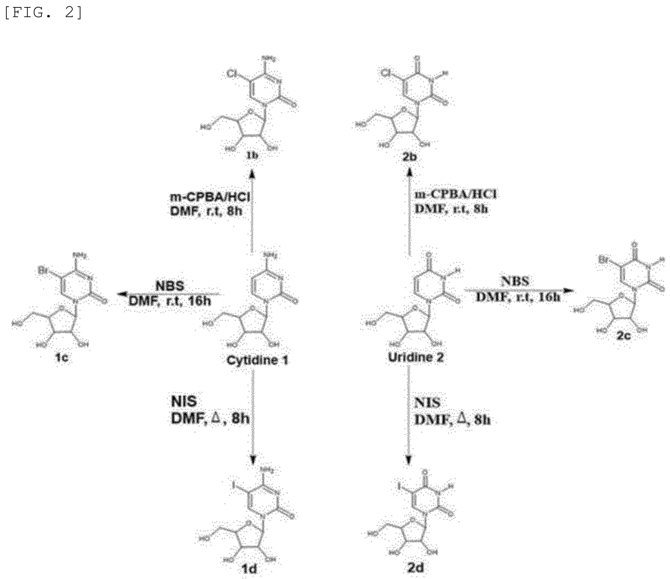

FIG. 2 is a schematic diagram showing the synthesis of C(5)-halogenated (Cl, Br, and I) cytidine and uridine ribonucleosides.
FIG. 3 is a diagram showing the process of synthesizing C(5)-halogenated cytidine ribonucleoside 5' -O-triphosphates by a one-pot synthesis method.
FIG. 4 is a diagram showing the process of synthesizing C(5)-halogenated uridine ribonucleoside 5' -O-triphosphates by a one-pot synthesis method.

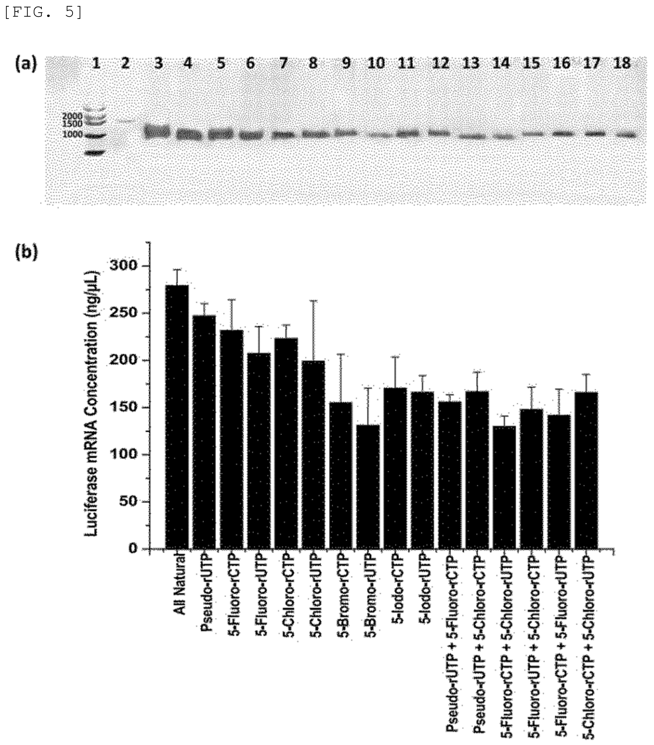

FIG. 5 is a schematic diagram briefly illustrating the experimental method for mRNA comprising C(5)-halogenated pyrimidine ribonucleotides according to the present invention and for increasing protein expression using the same.

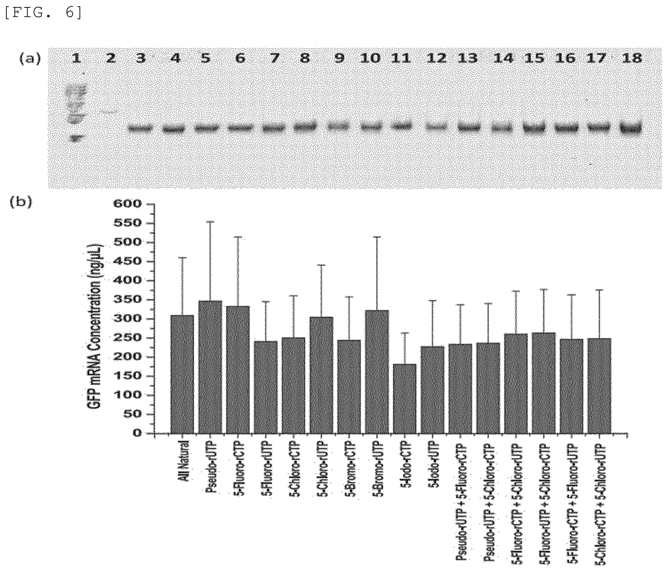

FIG. 6 shows the results of in vitro transcription (cap-dependent) of mRNA comprising C(5)-halogenated pyrimidine ribonucleotides: (a) Luciferase mRNA transcription bands. Lane 1: ladder, Lane 2: luciferase template, Lane 3: luciferase mRNA (all natural), Lane 4: luciferase mRNA (pseudo-rUTP included), Lane 5: luciferase mRNA (5-fluoro-rCTP included), Lane 6: luciferase mRNA (5-fluoro-rUTP included), Lane 7: luciferase mRNA (5-chloro-rCTP included), Lane 8: luciferase mRNA (5-chloro-rUTP included), Lane 9: luciferase mRNA (5-bromo-rCTP included), Lane 10: luciferase mRNA (5-bromo-rUTP included), Lane 11: luciferase mRNA (5-iodo-rCTP included), Lane 12: luciferase mRNA (5-iodo-rUTP included), Lane 13: luciferase mRNA (pseudo-rUTP and 5-fluoro-rCTP included), Lane 14: luciferase mRNA (pseudo-rUTP and 5-chloro-rCTP included), Lane 15: luciferase mRNA (5-fluoro-rCTP and 5-chloro-rUTP included), Lane 16: luciferase mRNA (5-fluoro-rUTP and 5-chloro-rUTP included), Lane 17: luciferase mRNA (5-fluoro-rCTP and 5-fluoro-rUTP included), Lane 18: luciferase mRNA (5-chloro-rCTP and 5-chloro-rUTP included). (b) Bar graph of transcription yield. The graph plots the yield of transcribed mRNA containing C(5)-halogenated pyrimidine nucleotides based on concentration (ng/µl). Each bar represents an average value obtained by performing NanoDrop analysis three times.

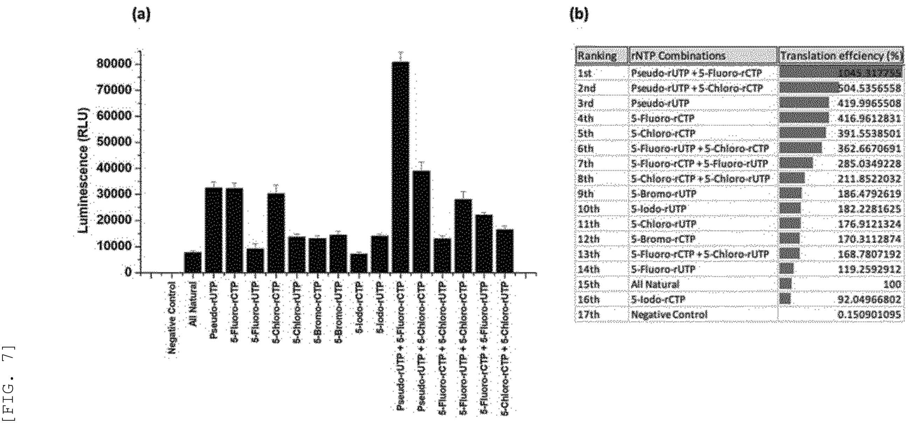

FIG. 7 shows (a) GFP mRNA transcription (cap-dependent). Lane 1: ladder, Lane 2: GFP template, Lane 3: GFP mRNA (all natural), Lane 4: GFP mRNA (pseudo-rUTP included), Lane 5: GFP mRNA (5-fluoro-rCTP included), Lane 6: GFP mRNA (5-fluoro-rUTP included), Lane 7: GFP mRNA (5-chloro-rCTP included), Lane 8: GFP mRNA (5-chloro-rUTP included), Lane 9: GFP mRNA (5-bromo-rCTP included), Lane 10: GFP mRNA (5-bromo-rUTP included), Lane 11: GFP mRNA (5-iodo-rCTP included), Lane 12: GFP mRNA (5-iodo-rUTP included), Lane 13: GFP mRNA (pseudo-rUTP and 5-fluoro-rCTP included), Lane 14: GFP mRNA (pseudo-rUTP and 5-chloro-rCTP included), Lane 15: GFP mRNA (5-fluoro-rCTP and 5-chloro-rUTP included), Lane 16: GFP mRNA (5-fluoro-rUTP and 5-chloro-rCTP included), Lane 17: GFP mRNA (5-fluoro-rCTP and 5-chloro-rUTP included), Lane 18: GFP mRNA (5-fluoro-rCTP and 5-chloro-rUTP included). (b) Bar graph of transcription yield. The graph plots the yield of transcribed mRNA containing C(5)-halogenated pyrimidine nucleotides based on concentration (ng/µl). Each bar represents an average value obtained by performing NanoDrop analysis three times.
FIG. 8 shows (a) translation yield of capped luciferase mRNA (error bars indicate standard deviation of three samples). (b) Ranking of translation yields according to rNTP combinations (the luminescence value represents the mean of three samples).
FIG. 9 is a graph showing the translation rate of IRES GFP mRNA.
FIGS. 10 to 12 respectively show verification of relative expression efficiencies of TLR7, IFN-α, and RIG-1 caused by intracellular introduction of mRNA comprising modified nucleotides according to the present invention for confirming immunogenicity.

### BEST MODE

Unless otherwise defined, all technical and scientific terms used in this specification have the same meanings as commonly understood by those skilled in the art to which the invention pertains. In general, the nomenclature used in this specification is well known and commonly employed in the art.

The invention relates to mRNA in which various halogens are introduced at the fifth carbon of a pyrimidine base. C(5)-halogenated (F, Cl, Br, I) pyrimidine ribonucleoside triphosphates were synthesized and were incorporated into mRNA during an in vitro transcription process. As a result, except for iodine (I), C(5)-halogenated pyrimidines having smaller size and higher electronegativity exhibited higher in vitro transcription efficiency (F > Cl > Br). Among the C(5)-halogenated pyrimidine ribonucleotides, mRNA comprising C(5)-halogenatedcytidine (5-F rCTP and 5-Cl rCTP) exhibited more prominent protein expression than C(5)-halogenated uridine-modified or unmodified mRNA. In particular, in mRNA into which fluorine (5-F rCTP) and chlorine (5-Cl rCTP) were introduced, proteins were expressed at efficiencies about four to five times higher than those of unmodified mRNA, similarly to pseudouridine (ψ). When pseudouridine (ψ) and 5-fluorocytidine (5-F rCTP) were simultaneously introduced into mRNA, thereby doubly incorporated, protein expression efficiency increased up to about ten-fold, demonstrating excellent effect. It was also confirmed that cap-dependent protein expression efficiency was much higher than cap-independent (IRES) expression in mRNA comprising C(5)-halogenated pyrimidine ribonucleotides. Therefore, based on these results, the invention can be usefully applied in the development of modified-mRNA-based therapeutics.

The invention provides an mRNA construct into which modified nucleotides are introduced using modified CTP and UTP in which the fifth carbon of the pyrimidine base of cytidine and uridine is substituted with a halogen, together with ATP (adenosine triphosphate) and GTP (guanosine triphosphate). Compounds in which the fifth carbon of the pyrimidine base of cytidine and uridine is substituted with a halogen have been used for antiviral purposes, but have not been employed for incorporation into an mRNA construct to enhance protein expression efficiency.

The invention also provides a composition for RNA synthesis comprising a modified nucleotide in which the fifth carbon of a pyrimidine base of cytidine or uridine is substituted with a halogen.

The composition for RNA synthesis may further comprise pseudouridine or methylpseudouridine.

The invention further provides a method for increasing protein expression efficiency using mRNA into which the modified nucleotide is introduced.

The method may be characterized in that protein expression efficiency is increased through cap-dependent translation.

The method may further comprise pseudouridine or methylpseudouridine, and particularly may comprise 5-fluoro cytidine triphosphate (5-F rCTP) represented by Chemical Formula 1 and pseudouridine, wherein the protein may be an antigen protein.

In the method for increasing protein expression efficiency using the mRNA construct, the mRNA construct may comprise ATP (adenosine triphosphate), GTP (guanosine triphosphate), CTP (cytidine triphosphate), and UTP (uridine triphosphate), and at least one of the ribonucleotides among CTP and UTP may have a halogen substituent at the fifth carbon of the pyrimidine base, particularly substituted with F or Cl, as represented by Chemical Formulas 1 to 4.

The halogen may be one or more selected from the group consisting of F (fluorine), Cl (chlorine), Br (bromine), and I (iodine), and more preferably one or more selected from the group consisting of F (fluorine), Cl (chlorine), and Br (bromine). Among CTP and UTP, at least one ribonucleotide in which a halogen (F, Cl, Br, or I) is substituted at the fifth carbon of the pyrimidine base was synthesized as shown in Example 1, and its specific chemical structures are represented by Chemical Formulas 1 to 8 (see Table 3).

The ribonucleotide of CTP in which the fifth carbon of the pyrimidine base is substituted with F (fluorine) is represented by Chemical Formula 1.

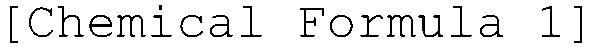

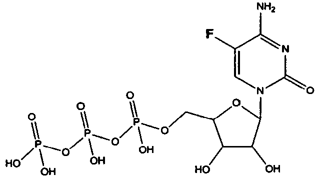

Another ribonucleotide of CTP in which the fifth carbon of the pyrimidine base is substituted with Cl (chlorine) is represented by Chemical Formula 3.

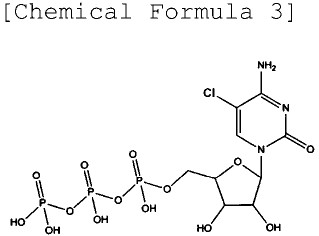

The method for increasing protein expression efficiency is characterized in that the level of protein expression is increased by cap-dependent translation, and it was confirmed that cap-dependent protein expression efficiency was much higher in mRNA comprising C(5)-halogenated pyrimidine ribonucleotides than in cap-independent (IRES) expression.

### EXAMPLE

### <Example 1> Synthesis of C(5)-halogenated pyrimidine ribonucleosides and 5' -O-triphosphates thereof

In the present invention, halogen derivatives (F, Cl, Br, and I) were introduced at the fifth carbon of uridine and cytidine ribonucleosides. Although various methods for halogenation at the C(5) position of pyrimidine nucleosides have been previously reported, in the present invention, halogen derivatives other than fluorine derivatives were also synthesized from commercially available cytidine and uridine ribonucleosides. These derivatives were confirmed and characterized by ¹H-NMR and ESI mass spectrometry. Triphosphates were attached to the C(5)-halogenated pyrimidine ribonucleosides, and RNA transcription was performed using T7 RNA polymerase. The Ludwig method, which does not require protection of the 2' and 3' hydroxyl groups of the nucleoside, was used to synthesize the C(5)-halogenated pyrimidine triphosphates. It was observed that the reaction time for triphosphate synthesis varied depending on the halogen introduced at the fifth carbon of the pyrimidine ribonucleoside. The triphosphate synthesis was found to be substrate-dependent, and in order to obtain a good yield for each nucleotide, the reaction time after the addition of pyrophosphate was extended from 2 to 3 hours to optimize the conditions. Each triphosphate compound was then purified using HPLC and confirmed by ³¹P-NMR analysis.

### <1-1> Reagents

All reagents for halogenation and triphosphate synthesis were commercially purchased and used without further purification. ¹H-NMR and ³¹P-NMR spectra were recorded using a Bruker AV 400 and 162 MHz spectrometer with DMSO-d₆ and D₂ O as solvents. UV-Vis spectra of the compounds were analyzed using a Cary Series UV-Vis spectrophotometer (Agilent Technology) with 1 cm path-length quartz cuvettes. After irradiation with ultraviolet light, absorbance changes were immediately measured. The luciferase plasmid was obtained from VectorBuilder (Vector ID: VB220810-1198vwd). All oligonucleotides were purchased from Cosmo Genetech, and the luciferase assay substrate ("Luciferase Assay System") was purchased from Promega. A 96-well polystyrene-treated cell culture plate was obtained from Corning, and 60 mL cell culture flasks were obtained from SPL Life Sciences. 2× TOPsimple^{™} DyeMIX-Tenuto PCR master mix, T7 RNA polymerase, and RNase inhibitor were purchased from Enzynomics. 3' -O-Mem7G(5' )ppp(5' )G RNA Cap Structure Analog (ARCA) and DNase I were purchased from New England Biolabs (NEB). The RNA purification kit "RNA Clean and Concentrator" was purchased from Zymo Research. Cellulose, ethanol, fetal bovine serum, penicillin/streptomycin, and trypsin were purchased from Sigma-Aldrich. Lipofectamine^{™} MessengerMAX^{™} transfection reagent, Opti-MEM, and DMEM were purchased from Thermo Fisher Scientific, and cells were cultured in a Heracell 240i CO₂ incubator. A Colibri+ LB 916 microvolume spectrophotometer and a Centro LB 960 microplate luminometer were purchased from Berthold Technologies.

### <1-2> Halogenation process and reactions

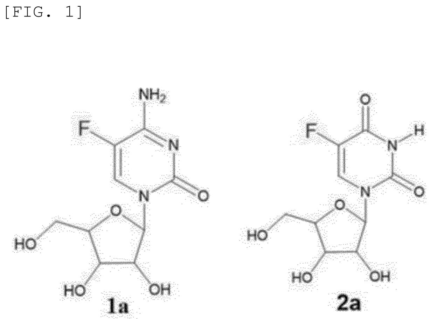

The reactions for cytidine and uridine were based on the structures of commercially available 5-fluorocytidine and 5-fluorouridine (FIG. 1). 5-Chlorocytidine and 5-chlorouridine (1b and 2b) were synthesized as follows. To synthesize cytidine 1b, 0.300 g (1.2 mM) of cytidine (1) was dissolved in 6 mL of DMF and stirred for 1 hour at room temperature in 0.5 M HCl. The temperature was then lowered to 0 °C, and 0.342 g (1.984 mM) of m-CPBA solution in DMF was added dropwise over 20 minutes, followed by raising the temperature to room temperature (15-25 °C). After stirring for 6 hours, the reaction mixture was extracted with EtOAc and water. Reaction progress was confirmed by TLC, and the aqueous layer was collected and dried using a rotary evaporator. The residue was made into a slurry and purified by silica gel column chromatography using 0-5% methanol/DCM, yielding the compound as a white solid powder (65%, 0.385 g).

¹H NMR (400 MHz, DMSO-d₆): δ 8.32 (s, 1H), 7.84 (s, 1H), 7.22 (s, 1H), 5.717 (d, 1H, J = 3.2 Hz), 5.386 (d, 1H, J = 4.8 Hz), 5.236 (t, 1H, J = 4.8 Hz), 5.498 (d, 1H, J = 5.6 Hz), 3.973-3.850 (m, 2H), 3.844 (t, 1H, J = 2.8 Hz), 3.726-3.689 (dq, 1H), 3.596-3.554 (dq, 1H), 3.341 (s, 8H), 2.506 (t, 4H, J = 1.6 Hz). MS (ESI, LRMS, m/z): calculated for C₉H₁₂ClN₃O₅ [M] = 277.66; found [M-2] = 275.6.

To synthesize uridine 2b, 0.300 g (1.2 mM) of uridine (2) was dissolved in 6 mL of DMF and stirred for 1 hour at room temperature in 0.5 M HCl. The temperature was lowered to 0 °C, and 0.3397 g (1.968 mM) of m-CPBA in DMF was added over 20 minutes, followed by increasing the temperature to room temperature. After 6 hours of stirring, the mixture was extracted with EtOAc and water, and the aqueous layer was collected, dried, and checked by TLC. The slurry was purified by silica gel column chromatography using 0-5% methanol/DCM, yielding a white solid powder (93%, 0.594 g).

¹H NMR (400 MHz, DMSO-d₆): δ 11.865 (s, 1H), 8.415 (s, 1H), 5.464 (d, 1H, J = 4.4 Hz), 5.441 (d, 1H, J = 5.3 Hz), 5.290 (t, 1H, J = 4.7 Hz), 5.085 (d, 1H, J = 5.4 Hz), 4.045 (dd, 1H, J = 4.8, 4.8 Hz), 3.998 (dd, 1H, J = 5, 5 Hz), 3.867 (t, 1H, J = 2.3 Hz), 3.721-3.672 (dq, 1H), 3.606-3.558 (dq, 1H), 3.336 (s, 22H), 2.506 (t, 18H, J = 1.6 Hz). MS (ESI, LRMS, m/z): calculated for C₉H₁₁ClN₂O₆ [M] = 278.03; found [M-2] = 276.6.

5-Bromocytidine and 5-bromouridine (1c and 2c) were synthesized as follows: To synthesize cytidine 1c, 0.300 g (1.2 mM) of cytidine (1) and 0.285 g (1.6 mM) of N-bromosuccinimide (NBS) were placed in a 200 mL round-bottom flask and dissolved in 5 mL of anhydrous DMF. The mixture was stirred overnight at room temperature under a nitrogen atmosphere. After completion, the solvent was removed by evaporation, and the slurry was purified by column chromatography using 0-5% methanol/DCM to afford a white solid powder (90%, 0.379 g).

¹H NMR (400 MHz, DMSO-d6): δ 8.398 (s, 1H), 7.850 (s, 1H), 7.004 (s, 1H), 5.714 (d, 1H, J = 3.2 Hz), 5.388 (d, 1H, J = 4.8 Hz), 5.239 (t, 1H, J = 4.8 Hz), 4.996 (d, 1H, J = 5.2 Hz), 4.108 (d, 1H, J = 5.2 Hz), 3.953 (dd, 2H, J = 5.2, 3.6 Hz), 3.845 (t, 1H, J = 2.4 Hz), 3.729-3.688 (dq, 1H), 3.591-3.543 (dq, 1H), 3.339 (s, 20H), 3.171 (d, 2H, J = 5.2 Hz), 2.506 (t, 10H, J = 2 Hz). MS (ESI, LRMS, m/z): calculated for C₉H₁₂BrN₃O₅ [M] = 321.00; found [M-2] = 319.6.

To synthesize uridine 2c, 0.300 g (1.2 mM) of uridine (2) and 0.285 g (1.6 mM) of N-bromosuccinimide (NBS) were dissolved in 5 mL of anhydrous DMF in a 200 mL round-bottom flask and stirred overnight under nitrogen at room temperature. After reaction completion, the solvent was evaporated, and the slurry was purified by column chromatography using 0-5% methanol/DCM to yield a white solid powder (70%, 0.2956 g).

¹H NMR (400 MHz, DMSO-d6): δ 11.827 (s, 1H), 8.490 (s, 1H), 5.730 (d, 1H, J = 4.4 Hz), 5.440 (s, 1H), 5.290 (s, 1H), 5.082 (s, 1H), 4.040 (d, 1H, J = 4 Hz), 3.988 (t, 1H, J = 4.4 Hz), 3.868 (t, 1H, J = 2.4 Hz), 3.694 (d, 1H, J = 12 Hz), 3.579 (d, 1H, J = 12 Hz), 3.341 (s, 6H), 2.506 (t, 3H, J = 2 Hz). MS (ESI, LRMS, m/z): calculated for C₉H₁₁BrN₂O₆ [M] = 321.98; found [M-1] = 320.6.

5-Iodocytidine and 5-iodouridine (1d and 2d) were synthesized as follows: To synthesize iodocytidine 1d, 0.300 g (1.2 mM) of cytidine (1) and 0.5399 g (2.4 mM) of N-iodosuccinimide were placed in a 200 mL round-bottom flask and dissolved in 6 mL of anhydrous DMF. The mixture was stirred at 60 °C for 8 hours under a nitrogen atmosphere. Reaction progress was monitored by TLC, the solvent was removed by rotary evaporation, and the residue was purified by column chromatography to obtain the product (87%, 0.4074 g).

¹H NMR (400 MHz, DMSO-d6): δ 8.412 (s, 1H), 7.811 (s, 1H), 6.616 (s, 1H), 5.707 (d, 1H, J = 3.6 Hz), 5.369 (d, 1H, J = 4.8 Hz), 5.224 (t, 1H, J = 4.8 Hz), 4.989 (d, 1H, J = 5.2 Hz), 3.941 (dd, 2H, J = 5.6, 4.4 Hz), 3.840 (t, 1H, J = 2.4 Hz), 3.718-3.680 (dq, 1H), 3.580-3.538 (dq, 1H), 3.341 (s, 19H), 2.506 (t, 6H, J = 1.6 Hz). MS (ESI, LRMS, m/z): calculated for C₉H₁₂IN₃O₅ [M] = 368.98; found [M-1] = 367.6.

To synthesize iodouridine 2d, 0.300 g (1.2 mM) of uridine (2) and 0.5399 g (2.4 mM) of N-iodosuccinimide were dissolved in 6 mL of anhydrous DMF in a 200 mL round-bottom flask and stirred at 60 °C for 8 hours under nitrogen. Reaction completion was confirmed by TLC, and the solvent was evaporated. The residue was purified by column chromatography to obtain a powder (73%, 0.3426 g).

¹H NMR (400 MHz, DMSO-d6): δ 11.690 (s, 1H), 8.482 (s, 1H), 5.722 (d, 1H, J = 4.4 Hz), 5.422 (d, 1H, J = 5.6 Hz), 5.265 (t, 1H, J = 4.8 Hz), 5.076 (d, 1H, J = 5.6 Hz), 4.034 (dd, 1H, J = 4.8, 4.8 Hz), 3.979 (dd, 1H, J = 4.8, 4.8 Hz), 3.863 (t, 1H, J = 2 Hz), 3.699-3.657 (dq, 1H), 3.592-3.551 (dq, 1H), 3.337 (s, 15H), 2.506 (t, 3H, J = 1.6 Hz). MS (ESI, LRMS, m/z): calculated for C₉H₁₁IN₂O₆ [M] = 369.97; found [M-1] = 368.5.

### <1-3> Synthesis of 5' -O-triphosphates of C(5)-halogen-substituted pyrimidine ribonucleosides

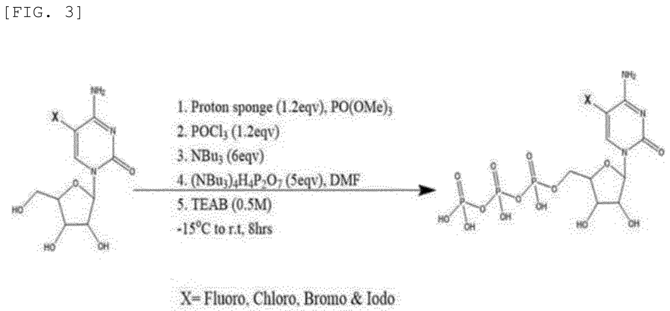

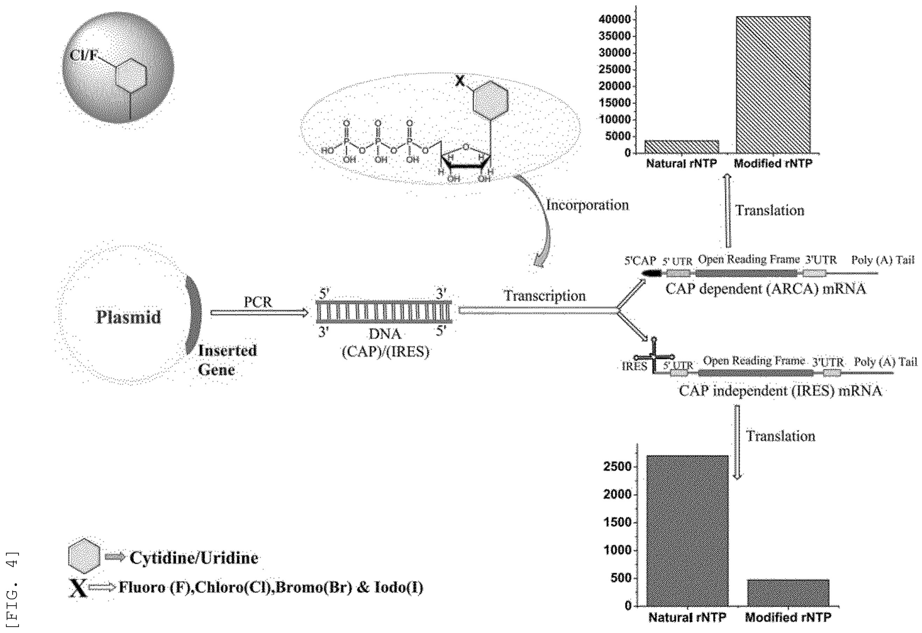

To prepare the 5' -O-triphosphates of the C(5)-halogen-substituted pyrimidine ribonucleosides, C(5)-halogenated cytidine ribonucleoside 5' -O-triphosphates were synthesized by a one-pot synthesis method (FIG. 3). C(5)-halogenated uridine ribonucleoside 5' -O-triphosphates were also synthesized by a one-pot synthesis method (FIG. 4). After the addition of pyrophosphate, the reaction time intervals and HPLC retention times for the C(5)-halogenated cytidine ribonucleosides are shown in Table 1 below, and those for the C(5)-halogenated uridine ribonucleosides are shown in Table 2 below.

**[Table 1]**

| No. | **5-Halogenated Cytidine Nucleoside** | **Reaction Time after Addition of Pyrophosphate (hours)** | **HPLC Retention Time (minutes)** |
|---|---|---|---|
| 1 | 5-Fluorocytidine | 2 | 20.10 |
| 2 | 5-Chlorocytidine | 3 | 27.56 |
| 3 | 5-Bromocytidine | 2.5 | 32.31 |
| 4 | 5-Iodocytidine | 3 | 33.34 |

**[Table 2]**

| No. | **5-Halogenated Uridine Nucleoside** | **Reaction Time after Addition of Pyrophosphate (hours)** | **HPLC Retention Time (minutes)** |
|---|---|---|---|
| 1 | 5-Fluorouridine | 0.05 to 2 | 23.46 |
| 2 | 5-Chlorouridine | 2.5 | 30.13 |
| 3 | 5-Bromouridine | 3 | 33.28 |
| 4 | 5-Iodouridine | 0.05 to 2 | 42.86 |

Specifically, proton sponge (1.2 equiv) and a free nucleoside (1 equiv) were placed in a reaction flask equipped with a magnetic stirrer and were dried overnight under high vacuum. The mixture (0.3 M) was dissolved in trimethyl phosphate and maintained at room temperature for 15 minutes until completely dissolved, then cooled to -15 °C. POCl₃ (1.2 equiv) was slowly added dropwise to the reaction slurry, during which a color change was observed, and the reaction was continued for 1.5 hours at the same temperature (-15 °C). Tri-n-butylamine (6 equiv) was then added slowly and stirred for 20 minutes, followed by the addition of a tri-n-butylammonium pyrophosphate solution (5.0 equiv) in DMF (0.5 M). The mixture was stirred for 5 minutes to 3 hours depending on the substrate (ribonucleoside). To obtain complete yield, the reaction time after pyrophosphate addition was extended for more than 2 hours before quenching with 0 °C water to optimize the reaction. A 5 M triethylammonium bicarbonate (Et₃NH₂CO₃, TEAB) solution (20 vol-equiv) was then added and stirred for 30 minutes while gradually warming from 0 °C to room temperature. The resulting solution was lyophilized. The crude product was purified by reversed-phase HPLC (prep pure C18 AQ 100 Å, 5 µm, 250 × 20 mm column) using 0.1 M triethylammonium acetate (TEAA buffer) as solvent A and MeCN (0-20 %) as solvent B over 60 minutes. The purified compound was lyophilized to obtain the triphosphate as a solid form. For confirmation, ³¹P-NMR spectra were measured using a Bruker AV-162 MHz spectrometer with D₂O as the solvent, and the chemical shifts (ppm) were observed around δ -23.65 to -23.10 (t, β-P), -11.90 to -11.75 (d, α-P), and -11.10 to -10.90 (d, γ-P), where t = triplet and d = doublet.

### <1-4> List of synthesized triphosphates

The list of triphosphates synthesized according to the invention is shown in Table 3 below.

**[Table 3]**

| No. | Name | Chemical Formula(NMR data) |
|---|---|---|
| 1 | 5-Fluorocytidine triphosphate (5-F rCTP) | 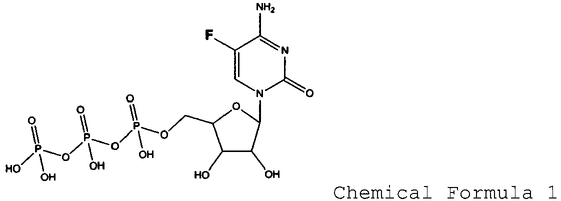 ³¹P NMR wastaken by using a Bruker AV-162 MHz spectrometer with D₂O as the solvent and gave chemical shift in ppm around; δ -23.53 to -23.28 (t, β-P), -11.72 to -11.60 (d, α-P), -11.06 to - 10.94 (d, γ-P). Where, t=triplet and d= doublet and followed by Electronspray ionization (ESI) mass Spectrometry. Calculated m/z for C₉H₁₅FN₃O₁₄P₃ ([M]): m/z 500.98; found ([M-1]): 499.60. |
| 2 | 5-Fluorouridine triphosphate (5-F rUTP) | 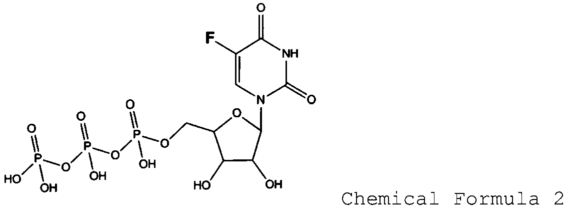 ³¹P NMR was taken by using a Bruker AV-162MHz spectrometer with D₂O as the solvent and gave chemical shift in ppm around; δ -23.50 to -23.25 (t, β-P), -11.85 to -11.58 (d, α-P), -11.03to - 10.72 (d, γ -P). Where, t=triplet and d= doublet and followed by Electronspray ionization (ESI) mass Spectrometry. Calculated m/z for C₉H₁₄FN₂O₁₅P₃ ([M]): m/z 501.96; found([M-1]): 500.50. |
| 3 | 5-Chlorocytidine triphosphate (5-Cl rCTP) | 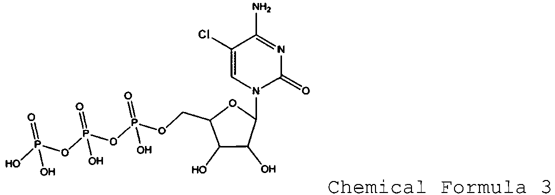 ³¹P NMR was taken by using a Bruker AV-162MHz spectrometer with D₂O as the solvent and gave chemical shift in ppm around; δ -23.56 to -23.31 (t, β-P), -11.98 to -11.68 (d, α-P), -11.06to - 10.94 (d, γ -P). Where, t=triplet and d= doublet and followed by Electronsprayionization (ESI) mass Spectrometry. Calculated m/z for C₉H₁₅ClN₃O₁₄P₃ ([M]): m/z 516.95; found: 516.50. |
| 4 | 5-Chlorouridine triphosphate (5-Cl rUTP) | 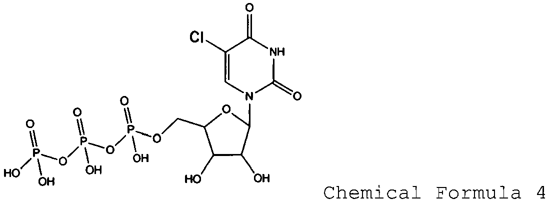 ³¹P NMR was taken by using a Bruker AV-162MHz spectrometer with D₂O as the solvent and gave chemical shift in ppm around; δ -23.57 to -23.33 (t, β-P), -11.85 to -11.73 (d, α-P), -11.06 to - 10.59 (d, γ-P). Where, t=triplet and d= doublet and followed by Electronspray ionization (ESI) mass Spectrometry. Calculated m/z for C₉H₁₄ClN₂O₁₅P₃ ([M]): m/z 517.93; found([M-1]): 516.50. |
| 5 | 5-Bromocytidine triphosphate (5-Br rCTP) | 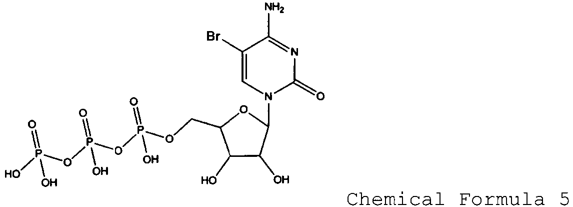 ³¹P NMR was taken by using a Bruker AV-162MHz spectrometer with D₂O as the solvent and gave chemical shift in ppm around; δ -23.52 to -23.27 (t,β-P), -11.98 to -11.85 (d, α-P), -11.00 to - 10.61 (d, γ -P). Where, t=triplet and d= doublet and followed by Electronspray ionization (ESI) mass Spectrometry. Calculated m/z for C₉H₁₅BrN₃O₁₄P₃ ([M]): m/z 560.90; found([M-1]): 559.5 |
| 6 | 5-Bromouridine triphosphate (5-Br rUTP) | 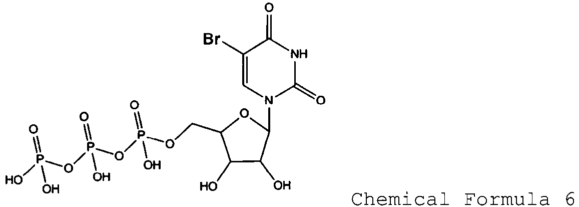 ³¹P NMR was taken by using a Bruker AV-162MHz spectrometer with D₂O as the solvent and gave chemical shift in ppm around; δ -23.54 to -23.30 (t,β-P), -11.89 to -11.56 (d, α-P), -11.06 to - 10.74 (d, γ -P). Where, t=triplet and d= doublet and followed by Electronspray ionization (ESI) mass Spectrometry. Calculated m/z for C₉H₁₄BrN₂O₁₅P₃ ([M]): m/z 561.88; found([M-1]): 560.50. |
| 7 | 5-Iodocytidine triphosphate (5-I rCTP) | 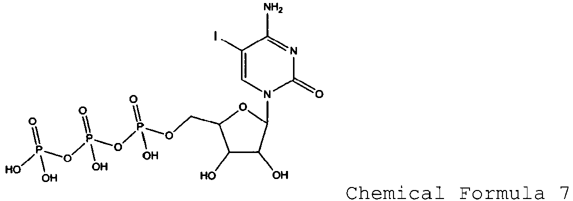 ³¹P NMR was taken byusing a Bruker AV-162MHz spectrometer with D₂O as the solvent and gave chemical shift in ppm around; δ -23.50 to -23.17 (t, β-P), -12.02 to -11.91 (d, α-P), -11.72 to - 10.61 (d, γ-P). Where, t=triplet and d= doublet and followed by Electronspray ionization (ESI) mass Spectrometry. Calculated m/z for C₉H₁₅IN₃O₁₄P₃ ([M]):m/z 608.88; found([M-1]): 607.40. |
| 8 | 5-Iodouridine triphosphate (5-I rUTP) | 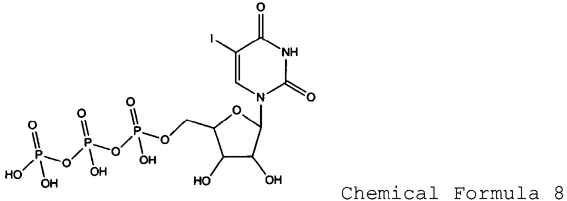 ³¹P NMR was taken by using a Bruker AV-162MHz spectrometer with D₂O as the solvent and gave chemical shift in ppm around; δ -23.54 to -23.29 (t, β-P), -11.93 to -11.80 (d, α-P), -11.72 to - 10.61 (d, γ-P). Where, t=triplet and d=doublet. And followed by Electronspray ionization (ESI) mass Spectrometry Calculated m/z for C₉H₁₄IN₂O₁₅P₃ ([M]):m/z 609.87; found([M-1]): 608.50. |

### <Example 2> In vitro transcription analysis of mRNA comprising C(5)-halogenated pyrimidines (cap-dependent mRNA platform)

To prepare modified mRNA, an in vitro transcription reaction was performed in which one of the four natural nucleoside triphosphates was replaced with the corresponding modified nucleoside triphosphate. In the mRNA transcribed in this way, single or double bases (uridine and cytidine) were substituted with C(5)-halogenated uridine or cytidine at all positions. A luciferase template (SI) was prepared from a luciferase plasmid replicated in E. coli, and modified luciferase mRNA was prepared through in vitro transcription. Upon confirmation by gel electrophoresis, a modified mRNA band (1.7 kb) was observed just below the template band (FIG. 6a). First, a cap-dependent in vitro transcription reaction was examined using the luciferase template (FIG. 6b). It was confirmed that the transcription yield of mRNA containing C(5)-halogenateduridine and cytidine triphosphates was approximately 10-50% lower compared to natural nucleotides. The reason for the lower yield of the modified nucleotides was assumed to be that the active site of T7 RNA polymerase is narrow for the recognition of modified nucleoside triphosphates, since natural nucleotides, rather than modified ones, are the preferred substrates of T7 RNA polymerase. In addition, in the case of C(5)-halogenated bases, it was observed that the transcription yield of the modified mRNA decreased in a regular manner up to the brominated nucleotides depending on the size and electronegativity of the halogen, and that the halogen derivatives having smaller atomic radius and higher electronegativity exhibited higher transcription yields. In the case of iodinated nucleotides, the mRNA transcription yield was slightly higher than that of brominated nucleotides in single-base modification of the genetic code, but in double-base modification, the iodinated nucleotides exhibited irregular transcription yield patterns. In the case of double-base modification of the genetic code, the overall transcription yield of the mRNA was slightly lower than that of single-base modification and showed irregularity.

### <Example 3> In vitro transcription analysis of mRNA comprising C(5)-halogenated pyrimidines (cap-independent mRNA platform)

A cap-independent in vitro transcription reaction was analyzed using C(5)-halogenated uridine and cytidine triphosphates, which were incorporated into mRNA containing an internal ribosome entry site (IRES) structure. The IRES is an RNA element that enables translation initiation in a cap-independent manner. For the analysis of cap-independent transcription, an IRES GFP template was used. Similar to the cap-dependent transcription analysis, all other transcription reagents were used in the same manner, except that the ARCA cap analog was not included. Since the secondary structure of the IRES within mRNA can affect translation efficiency, this experiment was conducted to confirm the effect of the modified rNTPs incorporated into mRNA containing the IRES structure and to compare it with natural rNTPs. Therefore, transcription was performed for all single- and double-modified IRES GFP mRNAs. As shown by gel electrophoresis analysis, all modified rNTPs were successfully transcribed, and the mRNA bands appeared immediately below the template band (FIG. 7a). Interestingly, according to the NanoDrop concentration analysis, the transcription efficiency of the modified rNTPs was relatively higher for single-base modifications than for double-base modifications in this case (FIG. 7b).

### <Example 4> In vitro translation analysis of mRNA comprising C(5)-halogenated pyrimidines (cap-dependent mRNA platform)

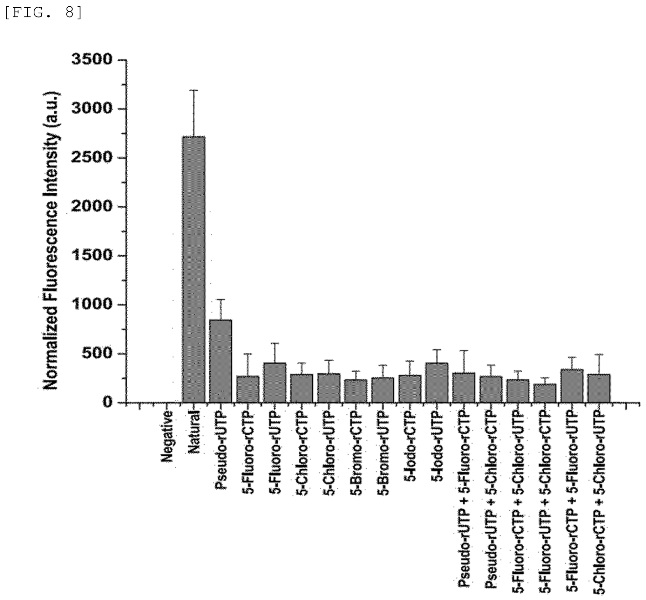

After the incubation period, the culture medium was removed, and the cells were lysed using 50 µL of 1× lysis buffer included in the luciferase substrate kit. For each sample, 100 µL of the luciferin substrate was placed into a 96-well white plate, and 20 µL of each cell lysate sample was added and mixed by pipetting. Luminescence was measured using a luminometer, and the results are shown in FIG. 8. FIG. 8(a) shows the translation yield, and FIG. 8(b) shows the ranking of translation efficiency according to the rNTP combinations. It was confirmed that, among the transcribed mRNAs, the double-modified integrated mRNAs (pseudouridine (ψ) + C(5)-fluorocytidine and pseudouridine (ψ) + C(5)-chlorocytidine) exhibited significantly enhanced translation efficiency compared with the other single- and double-modified mRNAs containing pseudouridine (ψ) (approximately 2.5-fold higher than pseudouridine alone). The single-modified mRNAs containing 5-Cl rCTP and 5-F rCTP showed approximately 1.1-fold higher translation efficiency than the mRNA containing pseudouridine (ψ) . Furthermore, it was confirmed that the mRNAs comprising C(5)-halogenated ribonucleotides having lower electronegativity and larger atomic size (iodocytidine/uridine and bromouridine) exhibited lower translation efficiency than the mRNA containing natural rNTPs.

### <Example 5> In vitro translation analysis of mRNA comprising C(5)-halogenated pyrimidines (cap-independent mRNA platform)

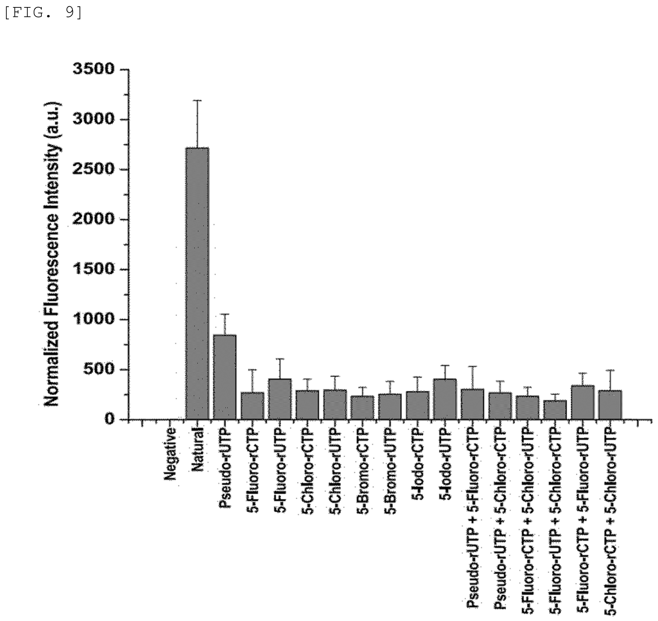

For cap-independent translation, transcribed IRES GFP mRNA, which did not contain a cap analog at the 5' end and included the EMCV IRES, was used. The effect of the mRNA into which C(5)-halogenated pyrimidines were incorporated was investigated. Interestingly, in the GFP expression analysis using both singly and doubly modified rNTPs, all modified mRNAs showed a markedly decreased translation efficiency, whereas the natural mRNA exhibited significant GFP expression (FIG. 9). The reason why this result was induced by the modification of the IRES is that the secondary structure of the IRES must be recognized by the initiation factors for translation. However, the IRES structure containing the C(5)-halogenated pyrimidines was altered in its secondary conformation due to the modification, differing from the natural structure, which may markedly reduce the translation efficiency of the target gene. In other words, the incorporation of the modified nucleotide may interfere with the formation of specific secondary structures required for the recognition and translation of the cellular translational machinery.

### <Example 6> Immunogenicity confirmation of mRNA comprising C(5)-halogenated pyrimidines (Verification of pattern recognition receptors and cytokine production for immunogenicity using reverse transcription polymerase chain reaction (RT-PCR))

To confirm the immunogenicity of the mRNA into which the modified nucleotide according to the invention was introduced when introduced into cells, the relative expression levels of TLR7, RIG-1, and IFN-α cytokine, which are pattern recognition receptors for exogenous single-stranded mRNA, were measured using macrophages (THP-1) and RT-PCR.

More specifically, the experiment for confirming the expression levels of the pattern recognition receptors (TLR7 and RIG-1) for exogenous single-stranded RNA and the production of IFN-α cytokine using THP-1 macrophages was performed using Apoptin mRNA into which the modified nucleotide was introduced, and the results were determined by reverse transcription polymerase chain reaction (RT-PCR). THP-1 monocytes were differentiated into macrophages using 200 ng/mL of phorbol 12-myristate 13-acetate (PMA). The modified Apoptin mRNA was transfected into the macrophages using Lipofectamine MessengerMAX (Thermo Fisher Scientific). After treatment for 6 hours, the cells were lysed, and total RNA was extracted using the Universal RNA Extraction Kit (Bioneer). After isolation of the total RNA, cDNA synthesis was performed using reverse transcriptase (Enzynomics) according to the manufacturer's instructions. In the next step, PCR reactions were carried out using gene-specific primers for TLR7, IFN-α, and RIG-1. For each amplified product, the cycle threshold (CT) value was normalized to the CT value of the internal control gene GAPDH to minimize inter-sample variability.

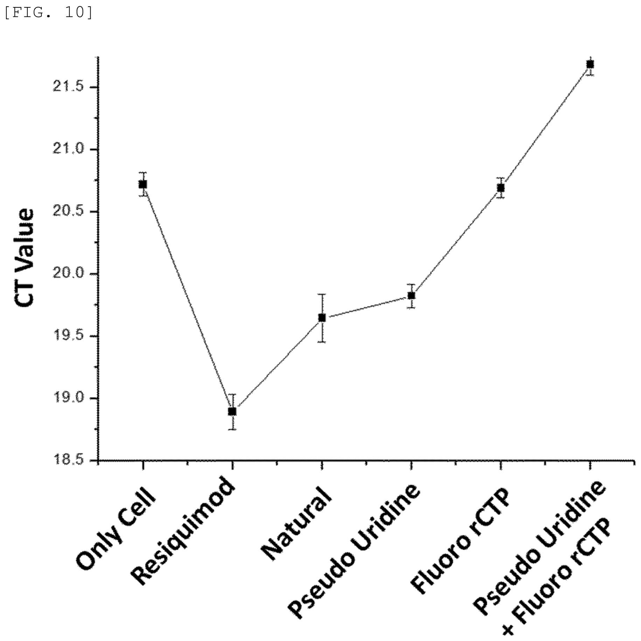

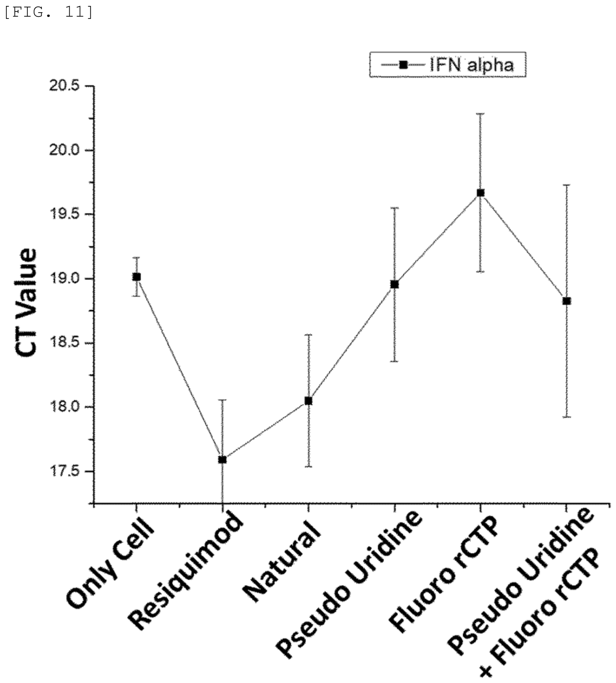

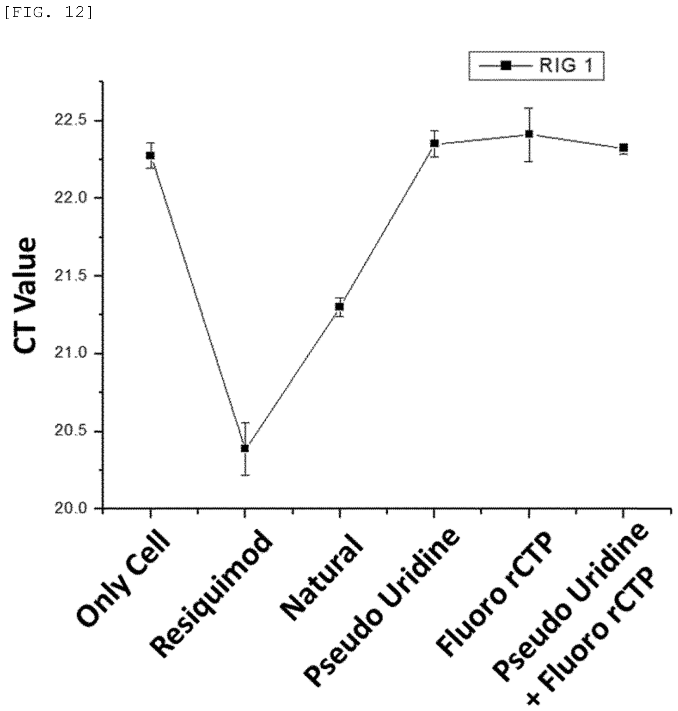

As a result, when the natural mRNA was introduced into the cells, the expression levels of TLR7, IFN-α, and RIG-1 were found to be high, whereas in the case of the mRNA containing pseudouridine, as previously reported, the expression levels were low. Furthermore, in the case of the mRNA into which the modified nucleotide 5-F CTP according to the invention was introduced alone or together with pseudouridine (double incorporation), it was confirmed that the expression levels of TLR7, IFN-α, and RIG-1 were similarly reduced, as in the mRNA containing pseudouridine. From these results, it was understood that the increased antigen protein expression of the mRNA containing 5-F CTP results from the reduction of excessive immune responses (FIGS. 10-12).

The above description of the invention is provided for illustrative purposes, and it will be understood by those skilled in the art that the invention may be easily modified into other specific forms without departing from the technical spirit or essential features of the invention. Accordingly, the embodiments described above should be understood as illustrative in all respects and not restrictive. For example, each component described as a single type may be implemented in a distributed form, and likewise, components described as distributed types may also be implemented in a combined form.

The scope of the invention is defined by the following claims, and all modifications or variations derived from the meaning and scope of the claims and equivalent concepts should be construed as being included within the scope of the invention.

### Industrial Applicability

The present invention relates to a method for increasing protein expression efficiency using an mRNA construct comprising C(5)-halogenated pyrimidine ribonucleotides. According to the invention, various C(5)-halogenated pyrimidine (cytidine/uridine) ribonucleotides were synthesized, and single- and double-base modified rNTPs were incorporated into cap-dependent and cap-independent mRNAs. As a result of confirming the transcription and translation efficiencies, it was found that fluorinated and chlorinated modified mRNAs exhibited 4-5 times higher translation efficiency than unmodified mRNA in the case of single-base modification, and that in the case of double-base modification, the combination of pseudouridine and 5-fluorocytidine showed an increase of about ten-fold or more compared with unmodified rNTPs. Accordingly, the invention can be effectively utilized in the development of therapeutic methods based on modified mRNA.

## Claims

1. An mRNA construct comprising a modified nucleotide in which the fifth carbon of a pyrimidine base of cytidine or uridine is substituted with a halogen.

2. The mRNA construct according to claim 1,
wherein the modified nucleotide is substituted with one selected from the group consisting of fluorine (F), chlorine (Cl), bromine (Br), and iodine (I).

3. The mRNA construct according to claim 1,
wherein the modified nucleotide is represented by one selected from the group consisting of the following Chemical Formulas 1 to 4:

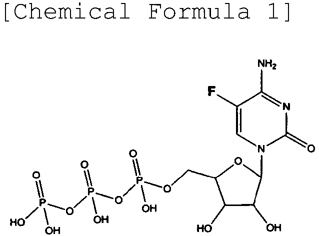

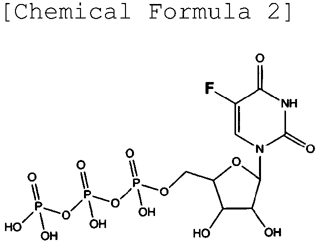

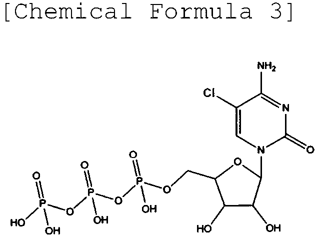

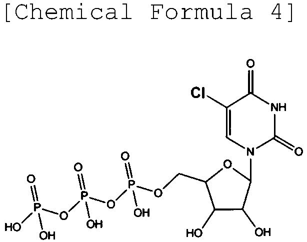

4. A composition for RNA synthesis comprising a modified nucleotide in which the fifth carbon of a pyrimidine base of cytidine or uridine is substituted with a halogen.

5. The composition for RNA synthesis according to claim 4,
wherein the modified nucleotide is represented by one selected from the group consisting of the following Chemical Formulas 1 to 4:

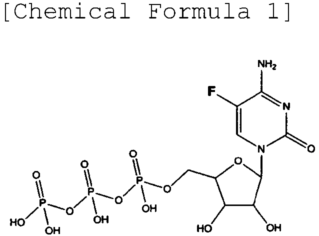

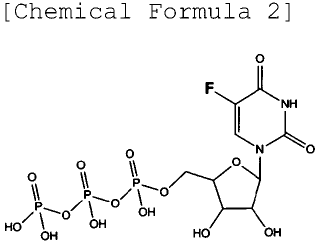

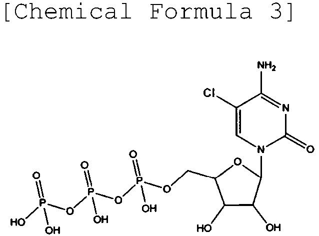

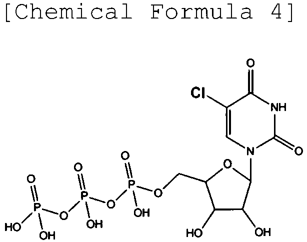

6. The composition for RNA synthesis according to claim 4,
wherein the composition further comprises pseudouridine or methylpseudouridine.

7. A method for increasing protein expression efficiency using the mRNA construct according to claim 1.

8. The method according to claim 7,
wherein the mRNA construct comprises a compound represented by one selected from the group consisting of the following Chemical Formulas 1 to 4:

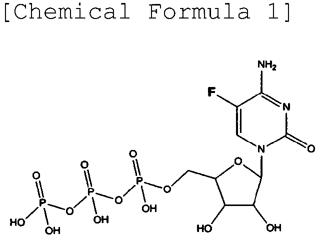

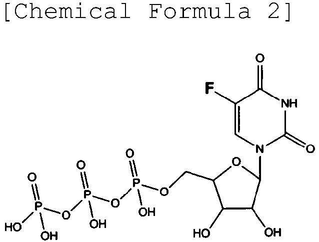

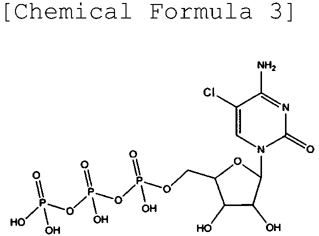

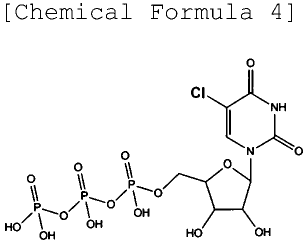

9. The method according to claim 7,
wherein the protein expression efficiency is increased through cap-dependent translation.

10. The method according to claim 7,
wherein the mRNA construct further comprises pseudouridine or methylpseudouridine.

11. The method according to claim 10,
wherein the mRNA construct comprises a compound represented by Chemical Formula 1 and pseudouridine:

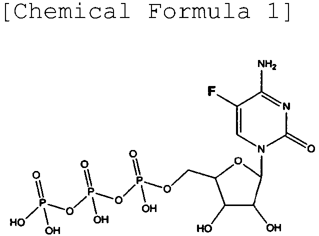

12. The method according to claim 7,
wherein the protein is an antigen protein.
